# EUROPÄISCHE PATENTSCHRIFT

(11) **EP 0 305 967 B1**
(45) Veröffentlichungstag und Bekanntmachung des Hinweises auf die Patenterteilung: **05.05.1993**
(21) Anmeldenummer: 88114130.3
(22) Anmeldetag: 30.08.1988
(51) Int. Cl.: A61K 37/66, A61K 39/395, A61K 47/42, A61K 37/02, C07K 15/14, C07K 15/26

(54) **Konjugate von Interferon alpha mit Immunglobulinen**
Conjugates of interferon alpha with immunoglobulins
Conjugués d'interféron alpha avec immunoglobulines

(30) Priorität: 02.09.1987 CH 3357/87
(43) Veröffentlichungstag der Anmeldung: 08.03.1989
(73) Patentinhaber: CIBA-GEIGY AG, 4002 Basel (CH)
(72) Erfinder: von Wussow, Peter, Dr., D-3017 Pattensen (DE)
(74) Vertreter: Zumstein, Fritz, Dr.

(56) Entgegenhaltungen:
- GB-A- 1 564 666
- CHEMICAL ABSTRACTS, Band 103, Nr. 3, 22. Juli 1985, Seite 449, Zusammenfassung Nr. 21010p, Columbus, Ohio, US; S.S. ALKAN et al.: "Antiviral and antiproliferative effects of interferons delivered via monoclonal antibodies"
- BIOLOGICAL ABSTRACTS, Band 78, Nr. 9, 1984, Seite 7803, Zusammenfassung Nr. 69267, Biological Abstracts Inc., Philadelphia, PA, US; G.R. FLANNERY et al.: "Immunomodulation: Natural killer cells activated by interferon-conjugated monoclonal antibody against human osteosarcoma",

## Beschreibung

Die vorliegende Erfindung betrifft neue Konjugate bestehend aus rekombinantem humanem Interferon alpha und menschlichem Immunglobulin, gewünschtenfalls verknüpft über einen organischen Brückenbildner, Verfahren zur Herstellung solcher Konjugate, pharmazeutische Zubereitungen und die Verwendung dieser Konjugate zur Herstellung von pharmazeutischen Präparaten zur Behandlung von viralen Infektionen und von Tumoren.

Humane Interferone alpha sind lösliche Proteine und Glykoproteine, die durch somatische Zellen, insbesondere immunkompetente Zellen, als Antwort auf eine Stimulierung ausgeschieden werden. Diese Interferone weisen eine Vielzahl biologischer Aktivitäten auf. Sie sind Botenmoleküle, die an der Regulation der Stoffwechsellage benachbarter Zellen beteiligt sind, in den Zellen lokal produziert werden und im Normalfall auch nur lokal wirksam sind. Sie verstärken oder unterdrücken dabei die Aktivität einer Nachbarzelle bezüglich einer spezifischen Eigenschaft.

Interferone alpha haben wie die meisten Cytokine ein Molekulargewicht im Bereich von 10'000 bis 40'000 Dalton. Sie schützen Zellen gegen eine Vielzahl von Viren, inhibieren das Zellwachstum in Kultur, beeinflussen die Immunantworten und erhöhen die Menge oder Aktivität gewisser spezifischer intrazellulärer Enzyme. Natürliches menschliches Interferon alpha (huIFNα) wird durch Leukozyten und lymphoblastoide Zellen hergestellt und umfasst mindestens ein Dutzend verschiedener Polypeptide, die sich in der Aminosäurenzusammensetzung und im Grad der Glykosylierung unterscheiden.

Menschliche Interferone alpha für therapeutische Anwendungen können durch Kultur stimulierter normaler menschlicher Zellen erhalten werden. Diese Quelle ist aber wegen der beschränkten Zugänglichkeit zu kultivierbaren menschlichen Zellen, der inhärenten Trennprobleme und der Kosten von geringer praktischer Bedeutung. Gewisse Interferone alpha können auch durch Kultur von kontinuierlichen menschlichen Zellinien gewonnen werden. Die am besten geeignete Methode zu ihrer, industriellen Herstellung ist jedoch die Kultivierung von gentechnologisch veränderten Bakterien, Pilzen oder Säugetierzellinien, denen die genetische Informationen zur Synthese des gewünschten Interferons durch rekombinante Gentechnik eingebaut worden ist. Diese Methode ermöglicht auch die Herstellung von Verbindungen, die sich von natürlichen Verbindungen unterscheiden und dadurch andere, bessere Eigenschaften für bestimmte Verwendungszwecke aufweisen.

Die therapeutische Anwendung von Interferon α ist nicht ohne Probleme. Sie werden normalerweise schnell abgebaut und/oder ausgeschieden. Bei einer systemischen Verabreichung ist es schwierig, die für eine antivirale oder anti-Tumor-Wirkung notwendige hohe Plasma-Konzentration aufzubauen und zu erhalten. Auf der anderen Seite entstehen unerwünschte Nebenwirkungen, die möglicherweise auf den Durchtritt der Interferone durch die Blut-Hirn-Schranke zurückzuführen sind. Es besteht deshalb ein ausgesprochenes Bedürfnis für Wege und Mittel, um einerseits therapeutisch wirksame hohe Interferon-Konzentrationen im Plasma zu erreichen, andererseits unerwünschte Nebenwirkungen in Grenzen zu halten oder zu verringern.

Das britische Patent GB 1 564 666 beschreibt Heterokomplexe aus Interferon, Immunglobulinen und Glutaraldehyd.

Es ist Ziel der vorliegenden Erfindung, Interferone alpha in einer Form zur Verfügung zu stellen, die die Verlängerung ihrer Aufenthaltsdauer und die Erhöhung ihrer Konzentration in Blutplasma und Geweben ermöglicht. Als besonders geeignet für die Lösung dieser Aufgabe erweisen sich Konjugate aus einem Interferon alpha und menschlichem Immunglobulin, verknüpft über einen organischen Brückenbildner, die überraschenderweise deutlich längere biologische Halbwertszeiten aufweisen als freie Interferone alpha. Ferner werden ein Verfahren zur Herstellung der Konjugate, pharmazeutische Zubereitungen und ihre Verwendung zur Behandlung von viralen Infektionen und von Tumoren beschrieben.

Ein humanes Interferon alpha ist ein lösliches Protein oder Glykoprotein, wie es von somatischen Zellen, beispielsweise von immunkompetenten Zellen, als Antwort auf eine Stimulierung synthetisiert und allenfalls ausgeschieden wird, um eine Stoffwechselumstellung benachbarter Zellen zu bewirken, beispielsweise um deren Wachstumseigenschaften zu beeinflussen, ferner eine verwandte Verbindung, die sich von einem natürlichen Interferon alpha durch Austausch, durch Fehlen oder durch Zusatz einer oder mehrerer Aminosäuren und/oder Kohlenhydratreste unterscheiden, oder ein Fragment einer solchen Verbindung, das die biologische Aktivität beibehält. Beispielsweise können eine, zwei, drei oder vier Aminosäuren des natürlichen Peptids durch andere Aminosäuren ausgetauscht sein oder ganz fehlen, eine, zwei, drei oder vier Aminosäuren zusätzlich vorhanden sein, z.B. am Aminoende des Peptids, einer oder zwei Kohlenhydratreste des natürlichen Glykoproteins durch andere Kohlenhydratreste ausgetauscht sein oder einer, zwei, drei oder alle Kohlenhydratreste fehlen. Ferner können verwandte Verbindungen beispielsweise auch aus Teilen zweier verschiedener Interferone alpha zusammengesetzt sein.

Beispiele für humane Interferone alpha nach dieser Definition sind die natürlich vorkommenden humanen Interferone alpha (IFNα), ferner durch rekombinante Gentechnik in Prokaryonten, in Eukaryonten oder Säugetierzellen hergestellte, mit den natürlichen Interferonen identische oder verwandte Verbindungen, wie rekombinantes IFNα, z.B. IFNα₁, IFNα₂, IFNα₃ etc. bis IFNα₁₆ beziehungsweise IFNα/A, IFNα/B, IFNα/D, IFNα/F oder IFNα/B-D-Hybride, ferner Fragmente der natürlichen oder rekombinanten Verbindungen, die die biologische Aktivität beibehalten.

Ein Immunglobulin ist ein humanes Immunglobulin (Antikörper), das zur Klasse der Immunglobuline A, D, E, G oder M, bevorzugt Immunglobulin G oder M, gehört. Dabei weist der variable Teil des Immunglobulins eine beliebige Spezifität auf. Vorzugsweise ist das Immunglobulin im Konjugat der Erfindung ein Gemisch von Antikörpern der gleichen Immunglobulin-Klasse, aber verschiedener, möglichst vielfältiger Spezifität, wie es aus Blut von gesunden Menschen gewonnen werden kann. Ein Immunglobulin im Konjugat der Erfindung kann aber auch ein polyklonaler oder monoklonaler menschlicher Antikörper spezifisch auf ein körperfremdes Antigen sein.

Ein Konjugat gemäss vorliegender Erfindung ist ein Hybridmolekül, in dem ein oder mehrere Interferone alpha und ein oder mehrere Immunglobuline kombiniert sind. Es handelt sich um einen chemischen Komplex, in dem die Kopplung über einen organischen Brückenbildner erfolgt. Die genannten chemischen Komplexe sind vorzugsweise unter physiologischen Bedingungen stabil.

Die genannten erfindungsgemässen Konjugate haben die Formel

Ck-Z-Ig (I),

worin Ck den Rest eines natürlichen oder rekombinanten humanen Interferons alpha, Ig den Rest eines menschlichen Immunglobulins und Z einen kovalent gebundenen, durch Oxo und/oder Imino substituierten Niederalkylen-dithio-niederalkylen-Rest darstellen, und worin Ck, Ig und/oder Z auch mehrfach vorkommen können.

Der Rest Ck ist ein- oder mehrwertig, z.B. zwei- oder dreiwertig, abhängig von der Anzahl der Wasserstoffatome und/oder Hydroxygruppen, die durch Bindungen zu Brückenbildnern Z ersetzt sind.

Der Rest Ig ist ein- oder mehrwertig, z.B. zwei-, drei-, vier-, fünf- oder sechswertig, abhängig von der Anzahl der Wasserstoffatome und/oder Hydroxygruppen, die durch Bindungen zu Brückenbildnern Z ersetzt sind.

Der Brückenbildner Z ist durch eine Amid-, Ester-, Amin-, Thioether- oder Thioester-Einfachbindung oder durch eine Kohlenstoff-Stickstoff-Doppelbindung kovalent an das Interferon und den Immunglobulinrest gebunden.

Z ist ein durch Oxo und/oder Imino substituierter Niederalkylen-dithioniederalkylen-Rest, kann aber auch aus 2-10 sich wiederholende Einheiten dieses Restes bestehen.

Der Ausdruck "Nieder" in Verbindung mit Niederalkylen bedeutet, dass diese Gruppe 1-7, bevorzugt 1-4, Kohlenstoffatome enthält. Niederalkylen hat vorzugsweise bis zu 7 Kohlenstoffatome und ist beispielsweise Methylen, Ethylen, 1,3-Propylen, 1,4-Butylen oder 1,6-Hexylen.

Der Brückenbildner Z ist mit dem Interferon und dem Immunglobulin-Rest beispielsweise über eine Amidbindung zwischen der endständigen Aminofunktion oder einer Seitenketten-Aminofunktion des Interferons oder Immunglobulins und einer Carboxyfunktion von Z, einer Stickstoff-Kohlenstoff-Einzel- oder Doppelbindung zwischen einer endständigen oder einer Seitenketten-Aminofunktion des Interferons oder Immunglobulins und einem Kohlenstoffatom von Z, durch eine Esterbindung zwischen einer Hydroxyfunktion einer Aminosäure oder eines Kohlenhydratrestes des Interferons oder Immunglobulins und einem Kohlenstoffatom von Z, durch eine Thioether- oder Thioester-Bindung zwischen einer Mercaptofunktion eines Cysteinrestes des Interferons oder des Immunglobulins und einem Kohlenstoffatom von Z verknüpft.

Ein solcher Brückenbildner Z ist der zweiwertige Rest Oxoniederalkylen-dithio-oxoniederalkylen, vorzugsweise Carbonyl-methylen-dithio-methylen-carbonyl, Carbonyl-ethylen-dithio-ethylen-carbonyl oder Carbonyl-propylen-dithio-propylen-carbonyl, Oxoniederalkylen-dithio-iminoniederalkylen, z.B. Carbonyl-methylen-, -ethylen- oder -propylen-dithio-propylen-carbonimidoyl, oder Iminoniederalkylen-dithio-iminoniederalkylen, z.B. Carbonimidoyl-propylen-dithio-propylen-carbonimidoyl oder Carbonimidoyl-butylen-dithio-butylen-carbonimidoyl. In solchen Brückenbildnern ist die Carbonylfunktion an das Interferon oder das Immunglobulin vorzugsweise über Amidbindungen und die Carbonimidoylfunktion vorzugsweise über Thioiminoesterbindungen geknüpft.

Die erfindungsgemässen Konjugate weisen im Vergleich zu den Interferonen alpha allein überraschenderweise eine wesentlich höhere Halbwertszeit der Verweildauer in Blutplasma und Geweben auf. Diese Beobachtung kann beispielsweise gemacht werden, indem Interferone alpha allein, erfindungsgemässe Konjugate und Gemische von nicht-konjugierten Interferonen alpha und Immunglobulin Versuchstieren, z.B. Mäusen, Ratten oder Kaninchen, intravenös, subkutan oder intramuskulär injiziert, in verschiedenen Zeitabständen Blut- oder Gewebeproben entnommen werden und deren Gehalt an Interferon bzw. Interferon Konjugaten bestimmt wird. Dabei zeigt sich, dass die Halbwertszeit der Verweildauer der erfindungsgemässen Konjugate im Vergleich zu Interferonen allein oder in Interferon Immunglobulin-Mischungen um einen Faktor 5 oder mehr erhöht ist.

Die erfindungsgemässen Konjugate können deshalb wie die entsprechenden Interferone alpha allein zur Behandlung von viralen Infektionen und/oder von Tumoren verwendet werden. Gegenüber den Interferonen selbst weisen die erfindungsgemässen Konjugate jedoch den Vorteil auf, dass sie wegen der längeren Verweilzeit im Organismus und insbesondere in der Blutbahn oder Gewebeteilen in deutlich geringeren Mengen eingesetzt werden können. Im weiteren ist zu erwarten, dass die erfindungsgemässen Konjugate bei direkter Applikation am Wirkungsort dort länger verweilen und damit weniger Nebenwirkungen, z.B. im Zentralnervensystem, hervorrufen.

Die Erfindung betrifft insbesondere Konjugate der Formel I, worin
Ck den Rest eines natürlichen oder rekombinanten humanen Interferons alpha darstellt, oder ein rekombinantes Protein oder Glykoprotein, in dem eine, zwei, drei oder vier Aminosäuren eines Interferons alpha ausgetauscht sind oder fehlen, zusätzlich eine, zwei, drei oder alle Kohlenhydratreste eines Interferons alpha fehlen, Teile zweier verschiedener Interferone alpha miteinander kombiniert sind oder nur der biologisch aktive Teil des Interferons alpha vorhanden ist,
Ig den Rest eines natürlichen humanen Immunglobulins darstellt, beispielsweise ein Immunglobulin G oder M aus Blut eines gesunden Menschen,
Z einen kovalent gebundenen, durch Oxo und/oder Imino substituierten Niederalkylen-dithio-niederalkylen-Rest darstellt
und worin Ck, Ig und/oder Z auch mehrfach, beispielsweise zwei- oder dreifach, vorkommen können.

Ganz besonders betrifft die Erfindung Konjugate der Formel I, worin
Ck den Rest eines natürlichen oder rekombinanten humanen Interferons darstellt, beispielsweise natürliches Interferon alpha, rekombinantes Interferon alpha, z.B. IFNα₁, IFNα₂, IFNα₃ bis IFNα₁₆ beziehungsweise IFNα/A, IFNα/B, IFNα/D, IFNα/F oder IFNα/B-D-Hybrid in glykosylierter oder unglykosylierter Form und mit gewünschtenfalls ausgetauschten, fehlenden oder einer bis vier zusätzlichen Aminosäuren,
Ig den Rest eines natürlichen humanen Immunglobulins darstellt, beispielsweise ein Immunglobulin G oder M aus Blut eines gesunden Menschen, und
Z einen kovalent gebundenen Oxoniederalkylen-dithio-oxoniederalkylen-Rest, z.B. Carbonyl-methylen-dithio-methylen-carbonyl, Carbonyl-ethylen-dithio-ethylen-carbonyl oder Carbonyl-propylen-dithio-propylen-carbonyl, Oxoniederalkylen-dithio-iminoniederalkylen-Rest, z.B. Carbonyl-ethylen-dithio-propylen-carbonimidoyl, oder Iminoniederalkylen-dithio-iminoniederalkylen, z.B. Carbonimidoyl-propylen-dithio-propylen-carbonimidoyl, darstellt In erster Linie betrifft die Erfindung Konjugate der Formel I, worin Ck den Rest eines natürlichen oder rekombinanten humanen Interferons alpha, z.B. IFNα_{2b} oder IFNα/B-D-Hybrid, Ig den Rest eines natürlichen humanen Immunglobulins G, beispielsweise wie es aus Blut eines gesunden Menschen gewonnen werden kann, und Z einen kovalent gebundenen, durch Oxo und/oder Imino substituierten Niederalkylen-dithio-niederalkylen-Rest, beispielsweise Carbonyl-ethylen-dithio-ethylen-carbonyl, Carbonyl-ethylen-dithio-propylen-carbonimidoyl oder Carbonimidoyl-propylen-dithio-propylen-carbonimidoyl, darstellen.

Zuallererst betrifft die Erfindung die in den Beispielen genannten Konjugate.

Die Erfindung betrifft auch ein Verfahren zur Herstellung von Konjugaten der Formel

Ck-Z-Ig (I),

dadurch gekennzeichnet, dass natürliches oder rekombinantes Interferon alpha mit menschlichem Immunglobulin und einem einen kovalent gebundenen, durch Oxo und/oder Imino substituierten Niederalkylen-dithio-niederalkylen-Rest bildenden Brückenbildner umgesetzt wird.

Die Konjugation erfolgt nach an sich bekannten Methoden.

Insbesondere betrifft die vorliegende Erfindung das genannte Verfahren, dadurch gekennzeichnet, dass
a) zur Herstellung eines Konjugates der Formel I, worin die Teile durch eine Amidbindung verknüpft sind, ein Teil enthaltend eine Aminogruppe mit dem komplementären Teil enthaltend eine Carbonsäure-Gruppe oder mit einem reaktiven Derivat davon kondensiert wird, oder
b) zur Herstellung eines Konjugates der Formel I, worin die Teile durch eine Disulfidbindung verknüpft sind, zwei komplementäre Teile, die beide eine Mercaptogruppe enthalten, mit einem milden Oxidationsreagens behandelt werden, oder
c) zur Herstellung eines Konjugates der Formel I, worin die Teile durch eine Disulfidbindung verknüpft sind, ein reaktives, substituierbares funktionelles Derivat der Mercaptogruppe eines Teils mit dem komplementären Teil enthaltend eine Mercaptogruppe substituiert wird.

Paare von Teilen, die zusammen gemäss Verfahren a) mit einer Amidbindung verknüpft werden können, sind beispielsweise ein Interferon und ein Immunglobulin, die beide eine freie Carboxygruppe bzw. eine freie Aminogruppe enthalten, ein Interferon und ein Immunglobulin substituiert durch einen oder mehrere Brückenbildner enthaltend eine freie Carboxy- und/oder Amino-Gruppe, ein Interferon substituiert durch einen oder mehrere Brückenbildner enthaltend eine freie Carboxy-und/oder Amino-Gruppe und ein Immunglobulin, oder ein Interferon und ein Immunglobulin, die beide einen Brückenbildnerteil enthaltend eine freie Carboxy-Gruppe beziehungsweise eine freie Aminogruppe tragen und diese beiden Teile zusammen einen Brückenbildner Z ergeben. Solche Paare von Teilen oder Mischungen von drei Teilen bestehend aus Interferon Immunglobulin und einem Brückenbildner enthaltend eine freie Carboxy- und eine freie Amino-Funktion werden mit einem Kondensationsmittel behandelt. Geeignete Kondensationsmittel sind jene, die überlicherweise in der Peptid-Chemie verwendet werden, z.B. Carbodiimide, z.B. N,N'-Dicyclohexylcarbodiimid, vorzugsweise wasserlösliche Carbodiimide wie N-Ethyl-N'-(3-dimethylaminopropyl)-carbodiimid oder N-Ethyl-N'-(3-N-morpholinopropyl)-carbodiimid, oder Isocyanide, z.B. 2-(N-Morpholino-ethyl)-isocyanid, gewünschtenfalls in Gegenwart eines tertiären Amins, z.B. Triethylamin, N-Methylmorpholin oder 4-Dimethylaminopyridin, und einer aktivierten, esterbildenden Hydroxy-Verbindung, z.B. N-Hydroxysuccinimid, N-Hydroxy-5-norbornen-2,3-dicarbonsäureimid oder 1-Hydroxybenztriazol. Andere geeignete Kondensationsmittel sind beispielsweise 2-Ethoxy-1-ethoxycarbonyl-1,2-dihydrochinolin oder aktivierte Phosphate wie Diphenylphosphorazidat oder Diethylphosphorcyanidat, gewünschtenfalls in Gegenwart eines tertiären Amins. Die Kondensation wird in einem wässrigen Lösungsmittel, das gegebenenfalls einen organischen oder anorganischen Puffer und/oder ein mischbares organisches Verdünnungsmittel enthält, z.B. ein Niederalkanol, z.B. tert-Butanol, ein Niederalkandiol, z.B. Glykol, Dimethylformamid, Acetonitril oder ähnliches, bei Temperaturen zwischen ungefähr -20°C und ungefähr +50°C, vorzugsweise zwischen 5°C und Raumtemperatur, durchgeführt.

Reaktive funktionelle Derivate von Teilen im Verfahren a) können in situ gebildet werden und sind beispielsweise reaktive Carbonsäure-Derivate, z.B. aktivierte Carbonsäureester, reaktive gemischte Anhydride, reaktive cyclische Amide, Isocyanate oder Isothiocyanate.

Aktivierte Ester von Carbonsäuren sind beispielsweise vom Vinylester-Typ, z.B. eigentliche Vinylester, Carbamoylvinylester oder 1-Niederalkylvinylester, Ester vom Amidin-Typ, z.B. N,N'-disubstituierte Amidinoester, welche durch Behandlung der entsprechenden Säure mit einem geeigneten N,N'-disubstituierten Carbodiimid erhalten werden können, beispielsweise mit N,N'-Dicyclohexylcarbodiimid, oder auch N,N-disubstituierte Amidinoester, geeignete Arylester, insbesondere durch elektronenanziehende Substituenten substituierte Phenylester, z.B. 4-Nitrophenyl-, 2,4,5-Trichlorphenyl-, Pentachlorphenyl- oder 4-Phenyldiazophenylester, Cyanmethylester, Thioester, insbesondere Nitrophenylthioester, oder vorzugsweise Amino- oder Amidoester, welche beispielsweise durch Behandlung der entsprechenden Säure mit einer N-Hydroxyamino- oder N-Hydroxyamido-Verbindung erhalten werden, beispielsweise mit N-Hydroxysuccinimid, N-Hydroxypiperidin, N-Hydroxyphthalimid, N-Hydroxy-5-norbornen-2,3-dicarbonsäureimid oder 1-Hydroxybenztriazol.

Reaktive gemischte Anhydride von Säuren sind beispielsweise Anhydride mit anorganischen Säuren, beispielsweise Säurehalogenide, insbesondere Säurechloride, Anhydride mit Kohlensäureniederalkylhalbestern, Anhydride mit dihalogenierten, insbesondere dichlorierten Phosphorsäuren oder Phosphorsäure-Derivaten, Anhydride mit starken Carbonsäuren, z.B. mit Trifluoressigsäure, oder Anhydride mit organischen Sulfonsäuren, z.B. mit Niederalkansulfonsäuren oder Arylsulfonsäuren, wie Methan- oder p-Toluolsulfonsäure.

Geeignete cyclische Amide sind insbesondere solche mit fünfgliedrigen aromatischen Diazaheterocyclen, beispielsweise Amide mit Imidazol oder Pyrazol, z.B. mit 3,5-Dimethylpyrazol.

Reaktive Carbonsäurederivate werden mit einem Reaktionspartner enthaltend eine freie Aminogruppe unter den gleichen Reaktionsbedingungen, wie oben für nichtaktivierte Teile beschrieben, umgesetzt, mit dem Unterschied, dass das Kondensationsmittel weggelassen werden kann. Vorzugsweise wird eine säurebindende Verbindung zugesetzt, beispielsweise ein Alkalimetallcarbonat oder -bicarbonat, z.B. Natrium- oder Kaliumcarbonat oder Natrium- oder Kaliumbicarbonat, üblicherweise zusammen mit dem entsprechenden Sulfat, oder eine organische Base, z.B. ein sterisch gehindertes Triniederalkylamin, z.B. Ethyldiisopropylamin.

Vorzugsweise sind die reaktiven Carbonsäuren Derivate des Brückenbildners, beispielsweise Dicarbonsäuredichloride, aktivierte Dicarbonsäurediester, Diisocyanate, z.B. Phenylen-1,4-diisocyanat oder 1-Methylphenylen-2,4-diisocyanat, oder Diisothiocyanate.

Die Ausgangsmaterialien für das Verfahren a) sind bekannt oder können nach bekannten Verfahren hergestellt werden, beispielsweise durch ein Verfahren wie es oben oder im folgenden beschrieben ist.

Paare von Teilen, die gemäss Verfahren b) oder c) eine Disulfid-Bindung ergeben können, sind z.B. ein Interferon und ein Immunglobulin, welche beide einen Brückenbildnerteil enthaltend eine Mercaptofunktion enthalten und worin die Brückenbildnerteile zusammen den Rest Z ergeben. Im Verfahren c) liegt die Mercapto-Gruppe eines der beiden Teile in Form eines reaktiven, substituierbaren funktionellen Derivates vor.

Geeignete Oxidationsmittel für das Verfahren b) der vorliegenden Erfindung sind insbesondere milde Oxidationsmittel, beispielsweise Jod, 1,2-Dijodethylen, o-Jodbenzoesäure, 1,10-Phenanthrolin und Kupfersulfat, Kaliumhexacyanoferrat(III) oder vorzugsweise Sauerstoff oder Luft. Die Reaktion wird in Wasser oder wässriger Pufferlösung bei einem pH zwischen ungefähr 3 und ungefähr 9, vorzugsweise nahe dem Neutralpunkt, durchgeführt, und zwar bei Temperaturen zwischen ungefähr -5°C und ungefähr Raumtemperatur. Das wässrige Reaktionsmedium enthält gewünschtenfalls ein mischbares organisches Lösungsmittel, beispielsweise einen Niederalkohol, z.B. tert-Butanol, Dimethylformamid, Acetonitril oder ähnliches, und/oder ein Alkalimetalljodid, beispielsweise Natrium- oder Kaliumjodid.

Ein reaktives, substituierbares funktionelles Derivat der Mercapto-Gruppe eines Teiles im Verfahren c) kann in situ gebildet werden und ist beispielsweise ein Sulfenylhalogenid, Sulfenamid, Sulfenylsulfon, Thiosulfat oder vorzugsweise ein gemischtes Disulfid.

Ein Sulfenylhalogenid ist beispielsweise ein Sulfenylchlorid oder ein Sulfenylbromid. Ein Sulfenamid ist beispielsweise von einem cyclischen Amin, einem Dicarbonsäureimid oder einem Heterocyclus abgeleitet und ist z.B. N-Sulfenylpiperidin, N-Sulfenylsuccinimid, N-Sulfenylphthalimid, N-Sulfenyl-5-norbornen-2,3-dicarbonsäureimid, 1-Sulfenylbenztriazol oder 1-Sulfenylimidazol.

Ein gemischtes Disulfid ist beispielsweise ein Disulfid mit einem aromatischen Thiol, z.B. mit Thiophenol substituiert durch Halogen, Nitro, Cyano oder Carboxy, z.B. mit o-Chlorthiophenol, 2,4-Dichlorthiophenol oder p-Nitrothiophenol, oder ein Disulfid mit einem heteroaromatischen Thiol, z.B. mit Pyridinthiol, vorzugsweise mit 2-Pyridinthiol, mit Mercaptopyridincarbonsäure, Chinolinthiol, 2-Mercaptobenzimidazol, 2-Mercaptobenzthiazol oder mit 2-Mercaptobenzoxazol.

Ein Sulfenylsulfon ist beispielsweise ein Niederalkylthiosulfonat, z.B. ein Methanthiosulfonat, oder ein Benzolthiosulfonat, das gewünschtenfalls durch Methyl, Nitro oder Halogen substituiert ist, z.B. p-Toluolthiosulfonat.

In einem Thiosulfat ist der organische Rest enthaltend die derivatisierte Mercapto-Funktion an den Schwefel gebunden. Vorzugsweise ist das Thiosulfat in anionischer Form, beispielsweise ein Alkalimetallthiosulfat, z.B. Natrium- oder Kaliumthiosulfat.

Besonders bevorzugt als reaktives, substituierbares funktionelles Derivat der Mercapto-Gruppe im Verfahren c) ist ein gemischtes Disulfid, insbesondere ein Disulfid mit 2-Pyridinthiol.

Die reaktiven funktionellen Derivate der Mercapto-Gruppe werden aus Verbindungen mit freier oder anderweitig derivatisierter Mercapto-Funktion nach den üblichen bekannten Methoden hergestellt.

In Verfahren c) werden der Reaktionspartner enthaltend eine freie Mercapto-Gruppe und der Reaktionspartner enthaltend ein reaktives, substituierbares funktionelles Derivat der Mercapto-Gruppe in Wasser oder vorzugsweise in wässrigem Puffer bei einem pH zwischen ungefähr 3 und ungefähr 9, vorzugsweise nahe dem Neutralpunkt, d.h. bei einem pH zwischen 6 und 8, bei Temperaturen zwischen ungefähr -20°C und ungefähr +50°C, vorzugsweise zwischen -5°C und Raumtemperatur, umgesetzt. Gewünschtenfalls enthält das wässrige Reaktionsmedium ein mischbares organisches Lösungsmittel, beispielsweise ein Niederalkanol, z.B. tert-Butanol, Dimethylformamid, Acetonitril oder ähnliches.

Die Ausgangsmaterialien für die Verfahren b) und c) sind bekannt oder können nach an sich bekannten Verfahren hergestellt werden, beispielsweise durch ein Verfahren, wie es oben oder im folgenden beschrieben ist. Ein substituiertes Interferon und ein substituiertes Immunglobulin, worin die Substituenten eine Mercapto-Gruppe tragen, werden beispielsweise aus dem Interferon beziehungsweise dem Immunglobulin durch Reaktion mit einer Verbindung enthaltend eine geschützte Mercapto-Gruppe und eine aktivierte Carbonsäurefunktion, beispielsweise einen aktivierten Ester wie oben unter Verfahren a) beschrieben, hergestellt. Die Schutzgruppe der Mercapto-Funktion wird dann durch an sich bekannte Verfahren abgespalten. Beispielsweise kann ein Disulfid, wie es oben als reaktives substituierbares funktionelles Derivat der Mercaptogruppe beschrieben ist, als Schutzgruppe während eines Verfahrens analog Verfahren a) verwendet werden und danach unter milden Reduktionsbedingungen gespalten werden, beispielsweise mit einem Thiol, z.B. 2-Mercaptoethanol, 3-Mercapto-1,2-propandiol, Thioglykolsäure, Thioglykolsäuremethylester oder 3-Mercaptopropionsäure, oder vorzugsweise mit einem Dithiol, z.B. Dithiothreitol oder Dithioerythritol, in wässriger Pufferlösung bei einem pH zwischen ungefähr 3 und ungefähr 6, vorzugsweise zwischen pH 4 und 5, bei Temperaturen zwischen ungefähr -5°C und ungefähr Raumtemperatur.

Ein substituiertes Interferon und ein substituiertes Immunglobulin, worin die Substituenten eine Mercapto-Funktion tragen, können beispielsweise auch aus einem Interferon beziehungsweise einem Immunglobulin durch Reaktion mit einem Iminothiolacton, beispielsweise mit 2-Iminothiolan, in wässriger Pufferlösung bei einem pH zwischen ungefähr 5 und ungefähr 9, vorzugsweise nahe dem Neutralpunkt, d.h. um pH 8, bei Temperaturen zwischen ungefähr -5°C und ungefähr Raumtemperatur, hergestellt werden.

Besonders bevorzugt ist ein Verfahren, worin ein Interferon oder ein Immunglobulin mit N-Succinimidyl-3-(2-pyridyldithio)-propionat gemäss Verfahren a) kondensiert, gleichzeitig das entsprechend ergänzende Immunglobulin oder Interferon mit 2-Iminothiolan in der oben beschriebenen Art und Weise umgesetzt und die beiden Teile gemäss Verfahren c) zu einer Dithiobindung verknüpft werden. Vorzugsweise werden die Reaktionsschritte ohne Isolierung der Zwischenprodukte durchgeführt.

Die Zwischenprodukte und Endprodukte der erfindungsgemässen Verfahren werden gewünschtenfalls gereinigt, beispielsweise durch Dialyse gegenüber Wasser oder Pufferlösungen, durch chromatograpische Methoden, vorzugsweise Gelchromatographie, Hochdruck-Flüssigchromatographie, Affinitätschromatographie oder Ionenaustauscherchromatographie, durch Ultrafiltration oder durch Ultrazentrifugation.

Die Ausgangsmaterialien und die Reaktionsbedingungen werden vorzugsweise so gewählt, dass jene in der Beschreibung als bevorzugt bezeichneten Verbindungen erhalten werden.

Die Konjugate der Erfindung können für die Behandlung von viralen Infektionen und Tumoren in der Form von pharmazeutischen Präparaten verwendet werden, welche eine wirksame Menge des Konjugats zusammen mit einer signifikanten Menge eines anorganischen oder organischen, festen oder flüssigen, pharmazeutisch verwendbaren Trägerstoffes enthalten.

Bevorzugt sind pharmazeutische Präparate zur parenteralen, beispielsweise intramuskulären, subkutanen oder intravenösen Verabreichung am Menschen. Solche Präparate sind isotonische wässrige Lösungen oder Suspensionen enthaltend die aktiven Konjugate zusammen mit einem Trägermaterial und, wenn erwünscht, Hilfsmittel, beispielsweise Stabilisatoren, Emulgiermittel, lösungsvermittelnde Stoffe, Salze für die Regulierung des pH und des osmotischen Druckes, Konserviermittel und/oder Netzmittel. Die pharmazeutischen Präparationen können nach an sich bekannten Methoden hergestellt werden, beispielsweise in einem Verfahren, worin die aktiven Konjugate und die pharmazeutisch verwendbaren Träger und Hilfsstoffe gemischt, gewünschtenfalls lyophilisiert und vor Verwendung gelöst werden.

Die Dosierung der pharmazeutischen Präparate enthaltend die aktiven Konjugate hängt von der zu behandelnden Krankheit, dem Körpergewicht, Alter und individuellen Zustand des Patienten gemäss Einschätzung des behandelnden Arztes und der Applikationsweise ab. Im Vergleich zur Dosis des entsprechenden freien Cytokins werden zwischen zwei- und ungefähr hundertfach geringere Mengen an gleichwertiger biologischer Aktivität eingesetzt. Beispielsweise wird ein Konjugat enthaltend Interferon alpha in ein bis drei täglichen Dosiseinheiten von 10⁴ bis 10⁶ IE (Internationale Einheiten der Interferon-Aktivität), vorzugsweise 10⁴ bis 10⁵ IE, verabreicht. Das Konjugat kann auch höher dosiert werden, beispielsweise derart, dass gleichviel biologische Aktivität wie beim freien Cytokin üblich eingesetzt wird. Dadurch wird bei einem höheren Prozentsatz der behandelten Patienten der gewünschte therapeutische Effekt des Cytokins erzielt.

Die folgenden Beispiele dienen zur Illustration der Erfindung, schränken deren Umfang jedoch in keiner Weise ein.

Die in den Beispielen verwendeten Abkürzungen haben folgende Bedeutungen:
- IE: Internationale Einheiten der IFN-Aktivität
- IFN: Interferon
- Ig: Immunglobulin
- IgG: Immunglobulin G
- PBS: phosphatgepufferte physiologische Kochsalzlösung
- rIFN: rekombinantes Interferon

### Beispiel 1: [rIFNα_{2b}][(C=NH)CH₂CH₂CH₂SSCH₂CH₂(C=O)][IgG]

1 mg polyklonaler Human-Antikörper IgG (Lyophilisat, Intraglobin F®, Biotest) wird in einem Eppendorf-Tübchen in 500 µl PBS, pH 7,2, gelöst. Zu der in einem Whirlmix geschüttelten IgG-Lösung werden 12 µl 1,6 mM 3-(2-Pyridyldithio)-propionsäure-N-hydroxysuccinimid-ester und 12 µl Ethanol zugetropft und die Mischung 30 Min. bei Raumtemperatur inkubiert. Danach wird über Nacht gegen PBS (3x Pufferwechsel) bei 4°C dialysiert.

10 µg rekombinantes Human-Interferon α_{2b} wird in einem Eppendorf-Tübchen in 500 µl PBS, pH 7,2, gelöst. 2-Iminothiolan wird bei 0°C in 1 M Triethanolaminhydrochlorid, pH 8,0, gelöst. 5 µl dieser Iminothiolan-Lösung werden zur IFN-Lösung gegeben und die Mischung 90 Min. bei Raumtemperatur inkubiert. Das Reaktionsgemisch wird viermal je 1 Std. bei 4°C gegen PBS dialysiert. Die IFN-Lösung wird mit der obigen IgG-Lösung gemischt, bei Raumtemperatur 2 Std. geschüttelt und über Nacht bei 4°C gehalten.

Das Konjugat wird von freiem IFN durch Ultrafiltration mittels eines Filters (durchschnittliche Ausschlussgrenze 50 000 Dalton) bei 4°C abgetrennt. Die beiden Fraktionen werden im CPE-Inhibitionsassay (Cytopathic Effect, siehe Ho und Enders, Proc. Natl. Acad. Sci. USA 45, 385-389 (1959)) auf IFN-Aktivität getestet. Aktives Konjugat findet sich im Ueberstand.

Zur Kontrolle wird die gesamte Reaktionssequenz mit radioaktiv markiertem ¹²⁵I-IFN wiederholt. Radioaktives Konjugat wird in den Fraktionen 1 bis 10 gefunden, radioaktives IFN in den Fraktionen 12 bis 25.

Die gleiche Reaktion wird auch mit rekombinantem Human-Interferon α/B-D-Hybrid (Ciba-Geigy AG, EP 205 404) und mit natürlichem Human-Interferon α (DRK Blutspendedienst Springe) anstelle von INFα_{2b} durchgeführt.

### Beispiel 2: [IFNα][(C=O)CH₂CH₂SSCH₂CH₂(C=O)][IgG]

2 mg polyklonaler Human-Antikörper IgG (Lyophilisat, Intraglobin F®, Biotest) und 100 µg (10⁷ IE) natürliches Human-Interferon α (DRK Blutspendedienst Springe) werden in je 1 ml PBS, pH 7,2, gelöst. Zu beiden Lösungen werden 18 µl 20 mM 3-(2-Pyridyldithio)-propionsäure-N-hydroxysuccinimidester und 82 µl PBS zugegeben, die Mischungen 30 Min. bei Raumtemperatur unter leichtem Schütteln inkubiert und über Nacht bei 4°C gegen je 500 ml PBS dialysiert. Die IFN-Lösung wird mit 0,1 % Albumin stabilisiert und bei 4°C aufbewahrt. Die IgG-Lösung wird mit 156 µl 0,1 M Natriumacetat (pH 4,5) auf ca. pH 6 gestellt, mit 3,8 mg Dithiothreitol versetzt und 20 Min. bei Raumtemperatur inkubiert, dann wieder über Nacht bei 4°C gegen PBS dialysiert. Die IFN-Lösung und die thiolierte IgG-Lösung werden gemischt, 2 Std. bei Raumtemperatur geschüttelt und über Nacht bei 4°C gehalten. Anschliessend wird das Konjugat wie in Beispiel 1 beschrieben abgetrennt.

### Beispiel 3: Verweilzeit des IFN-IgG-Konjugats in Mausserum

Je 0,4 ml einer Lösung des Konjugats von Beispiel 1 enthaltend 3 x 10⁴ IE werden drei Mäusen intramuskulär injiziert. Serum-Proben werden nach wenigen Minuten, 4, 8, 24, 48, 56 und 80 Stunden im CPE-Inhibitionsassay auf IFN-Aktivität untersucht. Dabei werden folgende Aktivitäten (log₁₀IE pro ml) gemessen:

| | 0 Std. | 4 Std. | 8 Std. | 24 Std. | 48 Std. | 56 Std. | 80 Std. |
|---|---|---|---|---|---|---|---|
| Maus 1 | 0,5 | 2,3 | 2,0 | 2,1 | 2,0 | 2,2 | 2,0 |
| Maus 2 | (2,0) | 2,2 | 2,2 | 2,2 | 2,0 | 2,2 | 2,2 |
| Maus 3 | 1,5 | 2,1 | 2,2 | 2,0 | 2,0 | 2,2 | 2,2 |
| Mittelwert | 1,2 | 2,2 | 2,1 | 2,1 | 2,0 | 2,2 | 2,1 |

Vergleichsweise fällt die IFN-Aktivität von intramuskulär injiziertem IFNα_{2b} allein (3 x 10⁴ IE) wesentlich stärker ab:

| | 0 Std. | 4 Std. | 24 Std. | 32 Std. | 56 Std. | 72 Std. | 80 Std. |
|---|---|---|---|---|---|---|---|
| Maus 1 | 0,7 | 2,3 | 0,7 | 0,5 | 0,5 | 0,5 | 0,5 |
| Maus 2 | 0,5 | 2,2 | 1,6 | 1,1 | 0,6 | 0,5 | - |
| Maus 3 | 0,5 | 0,8 | 2,1 | 0,9 | 0,5 | 0,5 | 0,5 |
| Maus 4 | - | 1,7 | 2,1 | 1,2 | - | 0,7 | 0,5 |
| Mittelwert | 0,6 | 2,0 | 1,9 | 1,0 | 0,5 | 0,5 | 0,5 |

Praktisch die gleichen Werte wie mit IFNα_{2b} allein werden gefunden, wenn Gemische von IFNα_{2b} und Intraglobin F (Human-IgG) injiziert werden.

### Beispiel 4: Allgemeine Vorschrift zur Herstellung von [Interferon α][(C=0)CH₂CH₂SSCH₂CH₂(C=0)[Ig]

Jeweils 2,5 mg Interferon alpha und Immunglobulin werden in 0,5 ml PBS pH 7,2 gelöst und mit je 100 µl einer frisch zubereiteten 3-(2-Pyridyldithio)-propionsäure-N-hydroxysuccinimidester-Lösung (4 mM) 30 Min. bei 25°C inkubiert. Eine PD-10 Gelfiltrations-Säule wird mit 20 ml einer Dithiothreitol-Lösung äquilibriert. Danach wird die Ig-Lösung auf die Säule aufgegeben und mit 6 ml Acetatpuffer pH 4,5 eluiert. Es werden Fraktionen a 500 µl aufgefangen und die Extinktion bei 280 nm vermessen. Proteinhaltige Fraktionen werden vereinigt, auf eine neue, mit PBS äquilibrierte PD-10 Gelfiltrations-Säule aufgetragen und mit Phosphatpuffer eluiert. Die Interferon-Lösung wird ebenfalls mit Phosphatpuffer über eine PD-10-Säule gelfiltriert, danach mit der thiolierten Ig-Lösung vermischt und 2 Std. bei Raumtemperatur und 20 Std. bei 4°C inkubiert. Das Reaktionsprodukt wird mit Polyacrylamidgel-Elektrophorese analysiert.

### Beispiel 5: Pharmazeutische Zusammensetzung

Lösungen enthaltend 10⁶ IE des IFNα-Konjugats von Beispiel 1 werden mit Human-Albumin bis zu einer Endkonzentration von 1 % versetzt, bei 4° gegen PBS dialysiert, durch einen bakteriologischen Filter sterilfiltriert und das Filtrat unter aseptischen Bedingungen in 10 Ampullen von 1 ml abgefüllt. Die Ampullen werden vorzugsweise in der Kälte, beispielsweise bei -18°C, aufbewahrt.

## Patentansprüche (Patentansprüche für folgende(n) Vertragsstaat(en): AT, BE, CH, DE, FR, GB, IT, LI, LU, NL, SE)

1. Konjugate der Formel
Ck-Z-Ig (I),
worin Ck den Rest eines natürlichen oder rekombinanten humanen Interferons alpha, Ig den Rest eines menschlichen Immunglobulins und Z einen kovalent gebundenen, durch Oxo und/oder Imino substituierten Niederalkylen-dithio-niederalkylen-Rest darstellen, und worin Ck, Ig und/oder Z auch mehrfach vorkommen können.

2. Konjugate gemäss Anspruch 1 der Formel I, worin Ck den Rest von rekombinantem IFNα_{2b}, Ig den Rest eines natürlichen humanen Immunglobulins G und Z Carbonimidoyl-propylen-dithio-ethylen-carbonyl darstellen.

3. Verfahren zur Herstellung von Konjugaten von humanem Interferon alpha mit menschlichem Immunglobulin gemäss Anspruch 1, dadurch gekennzeichnet, dass ein natürliches oder rekombinantes humanes Interferon alpha mit menschlichem Immunglobulin und mit einem einen kovalent gebundenen, durch Oxo und/oder Imino substituierten Niederalkylen-dithio-niederalkylenrest bildenden Brückenbildner umgesetzt wird.

4. Pharmazeutische Präparate enthaltend Konjugate gemäss Anspruch 1 der Formel I.

5. Verwendung von Konjugaten gemäss Anspruch 1 der Formel I zur Herstellung von pharmazeutischen Präparaten.

## Patentansprüche (Patentansprüche für folgende(n) Vertragsstaat(en): ES, GR)

1. Verfahren zur Herstellung von Konjugaten der Formel
Ck-Z-Ig (I),
worin Ck den Rest eines natürlichen oder rekombinanten humanen Interferons alpha, Ig den Rest eines menschlichen Immunglobulins und Z einen kovalent gebundenen, durch Oxo und/oder Imino substituierten Niederalkylen-dithio-niederalkylen-Rest darstellen, und worin Ck, Ig und/oder Z auch mehrfach vorkommen können, dadurch gekennzeichnet, dass natürliches oder rekombinantes Interferon alpha mit menschlichem Immunglobulin und einem einen kovalent gebundenen, durch Oxo und/oder Imino substituierten Niederalkylen-dithio-niederalkylen-Rest bildenden Brückenbildner umgesetzt wird.

2. Verfahren gemäss Anspruch 1 zur Herstellung von Konjugaten von rekombinantem IFNα_{2b}, natürlichem humanen Immunglobulin G und Carbon-imidoyl-propylen-dithio-ethylen-carbonyl.

3. Verfahren zur Herstellung von pharmazeutischen Präparaten enthaltend Konjugate gemäss Anspruch 1, dadurch gekennzeichnet, dass man die Konjugate mit einem pharmazeutischen Träger mischt.

## Claims (Claims for the following Contracting State(s): AT, BE, CH, DE, FR, GB, IT, LI, LU, NL, SE)

1. A conjugate of the formula
Ck-Z-Ig (I)
in which Ck is a residue of a natural or recombinant human interferon alpha, Ig is a residue of a human immunoglobulin, and Z is a covalently bonded oxo- and/or imino-substituted lower alkylene-dithio-lower alkylene radical, and in which Ck, Ig and/or Z also may occur several times.

2. A conjugate according to claim 1 of formula I in which Ck is a residue of recombinant IFNα_{2b}, Ig is a residue of a natural human immunoglobulin G, and Z is carbonimidoylpropylene-dithio-ethylene-carbonyl.

3. A process for the preparation of a conjugate of human interferon alpha with human immunoglobulin according to claim 1, which comprises reacting a natural or recombinant human interferon alpha with human immunoglobulin and with a bridge former that forms a covalently bonded oxo- and/or imino-substituted lower alkylene-dithio-lower alkylene radical.

4. A pharmaceutical composition comprising a conjugate according to claim 1 of formula I.

5. The use of a conjugate according to claim 1 of formula I for the preparation of a pharmaceutical composition.

## Claims (Claims for the following Contracting State(s): ES, GR)

1. A process for the preparation of a conjugate of formula
Ck-Z-Ig (I)
in which Ck is a residue of a natural or recombinant human interferon alpha, Ig is a residue of a human immunoglobulin, and Z is a covalently bonded oxo-and/or imino-substituted lower alkylene-dithio-lower alkylene radical, and in which Ck, Ig and/or Z also may occur several times, which comprises reacting natural or recombinant interferon alpha with human immunoglobulin and with a bridge former that forms a covalently bonded oxo- and/or imino-substituted lower alkylene-dithio-lower alkylene radical.

2. A process according to claim 1 for the preparation of a conjugate of recombinant IFNα_{2b}, natural human immunoglobulin G and carbonimidoyl-propylene-dithio-ethylene-carbonyl.

3. A process for the preparation of a pharmaceutical composition comprising a conjugate according to claim 1, which comprises mixing the conjugate with a pharmaceutical carrier.

## Revendications (Revendications pour l'(les) Etat(s) contractant(s) suivant(s): AT, BE, CH, DE, FR, GB, IT, LI, LU, NL, SE)

1. Conjugués de formule
Ck-Z-Ig (I)
dans laquelle Ck représente le radical d'un interféron alpha humain naturel ou recombinant, Ig le radical d'une immunoglobuline humaine et Z un radical alcoylène inférieur-dithio-alcoylène inférieur substitué par un oxo et/ou un imino, lié de façon covalente, et où Ck, Ig et/ou Z peuvent également se présenter en plusieurs exemplaires.

2. Conjugués selon la revendication 1 de formule I, dans laquelle Ck représente le radical de l'IFNα_{2b} recombinant, Ig le radical de l'immunoglobuline G humaine naturelle et Z un carbonimidoyl-propylène-dithio-éthylène-carbonyle.

3. Procédés de préparation de conjugués d'interféron alpha humain avec l'immunoglobuline humaine selon la revendication 1, caractérisés en ce qu'on fait réagir un interféron alpha humain naturel ou recombinant avec de l'immunoglobuline humaine et avec un formateur de pont formant un radical alcoylène inférieur-dithio-alcoylène inférieur substitué par un oxo et/ou un imino, lié de façon covalente.

4. Préparations pharmaceutiques contenant des conjugués selon la revendication 1 de formule I.

5. Application de conjugués selon la revendication 1 de formule I à la préparation de préparations pharmaceutiques.

## Revendications (Revendications pour l'(les) Etat(s) contractant(s) suivant(s): ES, GR)

1. Procédé de préparation de conjugués de formule
Ck-Z-Ig (I)
dans laquelle Ck représente le radical d'un interféron alpha humain naturel ou recombinant, Ig le radical d'une immunoglobuline humaine et Z un radical alcoylène inférieur-dithio-alcoylène inférieur substitué par un oxo et/ou un imino, lié de façon covalente, et où Ck, Ig et/ou Z peuvent également se présenter en plusieurs exemplaires, caractérisé en ce qu'on fait agir un interféron alpha humain naturel ou recombinant avec de l'immunoglobuline humaine et avec un formateur de pont formant un radical alcoylène inférieur-dithio-alcoylène inférieur substitué par un oxo et/ou un imino, lié de façon covalente.

2. Procédé selon la revendication 1 de préparation de conjugués d'IFNα_{2b} recombinant, d'immunoglobuline G humaine naturelle et de carbonimidoyl-propylène-diithio-éthylène-carbonyle.

3. Procédé de préparation de préparations pharmaceutiques contenant des conjugués selon la revendication 1, caractérisé en ce qu'on mélange les conjugués avec un support pharmaceutique.
